# EUROPEAN PATENT APPLICATION

(11) **EP 4 759 821 A1**
(43) Date of publication of application: **17.06.2026**
(21) Application number: 24851091.9
(22) Date of filing: 08.08.2024
(51) Int. Cl.: C07K 4/12, C07K 14/435, G01N 33/564

(54) **CITRULLINATED SETDB1 POLYPEPTIDE AND USE THEREOF IN DIAGNOSIS OF RHEUMATOID ARTHRITIS**

(30) Priority: 09.08.2023 CN 202310997528
(71) Applicant: Xiamen University, Xiamen, Fujian 361005 (CN); Xiamen Innodx Biotech Co., Ltd, Xiamen, Fujian 361022 (CN); Beijing Wantai Biological Pharmacy Enterprise Co., Ltd., Beijing 102206 (CN)
(72) Inventor: GE, Shengxiang, Xiamen, Fujian 361005 (CN); WENG, Lin, Xiamen, Fujian 361005 (CN); CHEN, Juan, Xiamen, Fujian 361005 (CN); LIU, Wei, Xiamen, Fujian 361005 (CN); DENG, Wen, Xiamen, Fujian 361005 (CN); HOU, Wangheng, Xiamen, Fujian 361005 (CN); SONG, Liuwei, Xiamen, Fujian 361022 (CN); QIAO, Shan, Beijing 102206 (CN); ZHANG, Jun, Xiamen, Fujian 361005 (CN); XIA, Ningshao, Xiamen, Fujian 361005 (CN)
(74) Representative: Lavoix
(86) International application number: PCT/CN2024/110642
(87) International publication number: WO 2025/031446

(57) **Abstract**

A method for diagnosing rheumatoid arthritis on the basis of the level of an anti-citrullinated SETDB 1 antibody, and a kit for use in the method. Provided are a polypeptide for the above-mentioned diagnosis of the level of an anti-citrullinated SETDB 1 antibody, and the use thereof in the diagnosis of rheumatoid arthritis.

## Description

### Cross-Reference to the Related Application

The present application is based on and claims priority to the Chinese application with application number CN 202310997528.X (filed on August 9, 2023). The disclosure of the Chinese application is incorporated herein by reference in its entirety.

### Technical Field

The present invention relates to immunological detection, and particularly to the field of immunological diagnostics. Specifically, the present invention provides a method for diagnosing rheumatoid arthritis based on anti-citrullinated SETDB1 antibody level, and a kit for use in the method. The present invention also provides a citrullinated SETDB1 polypeptide for the aforementioned diagnosis and its use in the diagnosis of rheumatoid arthritis.

### Background Art

Citrullination is a type of post-translational modification of proteins. It is a post-translational modification that occurs under the catalysis of a Ca+-dependent peptidylarginine deiminase (PAD) (Wang, R., et al., Gut stem cell necroptosis by genome instability triggers bowel inflammation. Nature, 2020. 580(7803): p. 386-390.). Because citrullination fundamentally alters protein structure and function, abnormal increases in citrullination in the body can lead to abnormally elevated levels of autoantibodies against citrullinated residue epitopes, thereby resulting in diseases. Relationships with citrullination have been reported in diseases such as rheumatoid arthritis, systemic lupus erythematosus, and tumors.

Among these, citrullination has been most frequently studied in rheumatoid arthritis. Rheumatoid arthritis is a systemic autoimmune disease of unknown etiology, affecting 0.5-1.0% of adults worldwide. It generally affects the synovial membrane of joints, often manifesting as joint pain and swelling. In severe cases, it can lead to joint deformities and even disability (MCINNES I B, SCHETT G. The pathogenesis of rheumatoid arthritis [J]. N Engl J Med, 2011, 365(23): 2205-2219.). Studies have found that arthritis is one of the 33 medical causes of motor-related functional loss in American adults (HOOTMAN J M, HELMICK C G, BRADY T J. A public health approach to addressing arthritis in older adults: the most common cause of disability[J]. Am J Public Health, 2012, 102(3): 426-433.). Therefore, rheumatoid arthritis can no longer be simply regarded as a common medical condition; it is also a public health issue that requires significant attention. Furthermore, some health economists have conducted research on the economic burden caused by rheumatoid arthritis. The results show that reducing risk factors or intervening early in the disease can significantly reduce costs compared to later hospitalizations and surgeries (LANES S F, LANZA L L, RADENSKY P W, et al. Resource utilization and cost of care for rheumatoid arthritis and osteoarthritis in a managed care setting: the importance of drug and surgery costs[J]. Arthritis Rheum, 1997, 40(8): 1475-1481.). In conclusion, controlling the onset and progression of rheumatoid arthritis requires "early detection, early diagnosis, and early treatment".

Studies have shown that the formation of autoantibodies is one of the characteristics that distinguishes rheumatoid arthritis from other inflammatory arthritis, such as psoriatic arthritis, reactive arthritis, and osteoarthritis (KOURILOVITCH M, GALARZA-MALDONADO C, ORTIZ-PRADO E. Diagnosis and classification of rheumatoid arthritis[J]. J Autoimmun, 2014, 48-49(26-30).). Currently, in laboratory tests, the diagnosis of rheumatoid arthritis mainly relies on rheumatoid factors (RFs) and anti-citrullinated protein antibodies (ACPAs). Rheumatoid factors are produced by the body due to the stimulation of persistent infection by foreign pathogens such as viruses and mycoplasma. Therefore, positive results can also be detected in patients with other immune diseases, patients with chronic infections, and 5% of healthy individuals, and it is generally used as an indicator for early disease screening. Among anti-citrullinated protein antibodies, anti-cyclic citrullinated peptide (CCP) antibodies have become the most commonly used clinical diagnostic label due to their high specificity. Studies have shown that this marker has a specificity of up to 90%, but a sensitivity of only about 70% (Wegner, N., Lundberg, K., Kinloch, A., Fisher, B., Malmström, V., Feldmann, M., & Venables, P. J. (2010). Autoimmunity to specific citrullinated proteins gives the first clues to the etiology of rheumatoid arthritis. Immunological reviews, 233(1), 34-54. https://doi.org/10.1111/j.0105-2896.2009.00850.x). Due to the issue of low sensitivity, it often leads to missed diagnoses in patients, and there is an urgent need to identify new diagnostic labels for rheumatoid arthritis. Furthermore, CCP is an artificially designed cyclic citrullinated peptide, which can only be used for the clinical diagnosis of rheumatoid arthritis and has little significance for exploring the disease mechanism.

In summary, citrullination is involved in the onset and progression of rheumatoid arthritis (RA). Therefore, naturally occurring citrulline markers in RA patients hold promise as high-performance markers for diagnosis and mechanistic exploration. Screening for naturally occurring high-level citrulline markers in patients can not only address the current problem of limited clinical markers but also potentially uncover key factors in the pathogenesis of RA.

### Contents of the Invention

Histone-lysine N-methyltransferase SETDB1 (SETDB1) belongs to the Suppressor of Variegation 3-9 (SUV39) protein family and is a member of the SET domain-containing protein lysine methyltransferases (PKMTs) involved in epigenetic regulation. It enables trimethylation of histone H3 at the ninth lysine residue (H3K9me3). Current research primarily considers SETDB1 to be closely associated with cancer. Elevated SETDB1 expression levels have been observed and implicated in tumorigenesis and progression in many types of cancers, including colorectal cancer, liver carcinoma, gastric cancer, and lung cancer. SETDB1 may serve as one of combined targets for tumor therapy; for example, inhibiting SETDB1 can enhance the sensitivity of cetuximab in colorectal cancer, overcoming its resistance. In melanoma cells with high SETDB1 expression, treatment with SETDB 1 inhibitors in combination with BAKR and MEK inhibitors can effectively kill cells resistant to targeted therapy.

The inventors of the present application screened serum samples from rheumatoid arthritis (RA) patients for citrulline markers innovatively by constructing chemical derivatives, and for the first time discovered a citrullinated SETDB1 epitope polypeptide. This epitope polypeptide exhibits high levels of autoantibodies in RA patients, demonstrating diagnostic value in rheumatoid arthritis. Therefore, the inventors provide a diagnostic method for RA based on the detection of anti-citrullinated SETDB1 antibody level, and a citrullinated SETDB1 polypeptide used for the method, which can effectively reduce the missed diagnosis rate of early-stage RA patients and may even outperform current clinical diagnostic markers.

### Isolated Polypeptide or Variant thereof

In a first aspect, the present invention provides an isolated polypeptide or variant thereof, wherein the polypeptide is composed of at least 11 consecutive amino acid residues of the SETDB 1 protein MBD domain;
wherein, at least one of the consecutive amino acid residues is an arginine residue, and at least one of the arginine residues is citrullinated;
wherein, the variant differs from the polypeptide from which it derived only in a substitution (e.g., a conservative or non-conservative substitution; e.g., a conservative substitution) of one or more (e.g., 1, 2, 3, 4, 5, 6, 7, 8, or 9) amino acid residues, and retains a biological function (e.g., an activity of being recognized and bound by an anti-citrullinated SETDB1 antibody) of the polypeptide from which it derived.

As used herein, the biological function of the polypeptide or variant thereof of the present invention includes, but is not limited to, an activity of being used as an epitope polypeptide and recognized and bound by an anti-citrullinated antibody (e.g., an anti-citrullinated SETDB1 antibody).

As used herein, when referring to the amino acid position of the SETDB1 protein MBD domain, it is described by referring to the sequence as set forth in SEQ ID NO: 1. For example, the expression "amino acid residue at the position 2 of the SETDB1 protein MBD domain" refers to the amino acid residue at the position 2 of the sequence as set forth in SEQ ID NO: 1 and its corresponding position. The corresponding position refers to the position in the sequence to be compared that is equivalent to a specific amino acid position in SEQ ID NO: 1 when the sequence to be compared is optimally aligned with SEQ ID NO: 1 (i.e., to obtain the highest percentage of identity).

In some embodiments, all arginine residues in the consecutive amino acid residues are citrullinated (i.e., converted into citrullines).

In some embodiments, the isolated polypeptide comprises an arginine residue at least at one (e.g., 1, 2, 3, 4, 5, 6, 7, 8, or all 9) of amino acid positions corresponding to the following positions of the SETDB1 protein MBD domain: 2, 16, 17, 21, 22, 23, 26, 43, and/or 50; and at least one (e.g., 1, 2, 3, 4, 5, 6, 7, 8, or all 9) of the arginine residues is citrullinated.

In some embodiments, the isolated polypeptide comprises at least amino acid residues at the positions 16-26 of the SETDB1 protein MBD domain; wherein at least one of the consecutive amino acid residues at the positions 16-26 is an arginine residue, and at least one of the arginine residues is citrullinated. In some embodiments, all arginine residues in the consecutive amino acid residues at the positions 16-26 are citrullinated.

In some embodiments, the isolated polypeptide comprises an arginine residue at least at one (e.g., 1, 2, 3, 4, 5, or all 6) of amino acid positions corresponding to the following positions of the SETDB1 protein MBD domain: 16, 17, 21, 22, 23, and/or 26; and at least one (e.g., 1, 2, 3, 4, 5, or all 6) of the arginine residues is citrullinated. In some embodiments, all of the arginine residues are citrullinated.

In some embodiments, the isolated polypeptide comprises the sequence as set forth in SEQ ID NO: 8 at the positions 16-26 corresponding to the SETDB1 protein MBD domain.

In some embodiments, the isolated polypeptide further comprises an arginine residue at least at one (e.g., 1, 2, or all 3) of amino acid positions corresponding to the following positions of the SETDB1 protein MBD domain: 2, 43, and/or 50; and at least one (e.g., 1, 2, or all 3) of the arginine residues is citrullinated.

In some embodiments, the isolated polypeptide is composed of a consecutive amino acid residues selected from the following: amino acid residues at the positions 5-27, 8-27, 12-27, 14-27, 16-27, 16-26, 1-27, or 5-32 of the SETDB1 protein MBD domain; wherein at least one of these consecutive amino acid residues is an arginine residue, and at least one of these arginine residues is citrullinated. In some embodiments, all arginine residues in the consecutive amino acid residues are citrullinated.

In some embodiments, the isolated polypeptide comprises an arginine residue at least at one (e.g., 1, 2, 3, 4, 5, or all 6) of amino acid positions corresponding to the following positions of the SETDB1 protein MBD domain: 16, 17, 21, 22, 23, and/or 26; and at least one (e.g., 1, 2, 3, 4, 5, or all 6) of these arginine residues is citrullinated.

In some embodiments, the isolated polypeptide further comprises an arginine residue at the position 2 of the SETDB1 protein MBD domain, which is optionally citrullinated.

In some embodiments, the variant of the present invention has the exact same citrulline characteristics as the polypeptide from which it is derived, i.e., has the same number and positions of citrulline residues.

In some embodiments, the variant, compared to the polypeptide from which it is derived, does not comprise amino acid substitutions at amino acid positions corresponding to the following positions of the SETDB1 protein MBD domain: 16, 17, 21, 22, 23, and 26. That is, the variant is identical to the polypeptide from which it is derived at amino acid positions corresponding to the positions 16, 17, 21, 22, 23, and 26 of the SETDB1 protein MBD domain. In some embodiments, the variant further does not comprise amino acid substitutions at amino acid positions corresponding to the positions 2, 43, and/or 50 of the SETDB1 protein MBD domain. That is, the variant is further identical to the polypeptide from which it is derived at amino acid positions corresponding to the positions 2, 43, and/or 50 of the SETDB1 protein MBD domain.

In some embodiments, the variant of the present invention differs from the polypeptide from which it is derived only in a substitution (e.g., a conservative or non-conservative substitution) of one, two, three, or four amino acid residues, and retains a biological function (e.g., an activity of being recognized and bound by an anti-citrullinated SETDB1 antibody) of the polypeptide from which it is derived.

In some embodiments, the polypeptide and variant thereof of the present invention comprises arginine residues at amino acid positions corresponding to the positions 16, 17, 21, 22, 23, and 26 of the SETDB1 protein MBD domain, and at least one (e.g., 1, 2, 3, 4, 5, or all 6) of these arginine residues is citrullinated.

In some embodiments, the polypeptide and variant thereof of the present invention comprises citrullinated arginine residues (i.e., citrullines) at amino acid positions corresponding to the positions 16, 17, 21, 22, 23, and 26 of the SETDB1 protein MBD domain.

In some embodiments, the polypeptide and variant thereof further comprise an arginine residue at amino acid position corresponding to the position 2 of the SETDB1 protein MBD domain, which is optionally citrullinated.

In some embodiments, the polypeptide and variant thereof further comprises an arginine residue at position corresponding to the amino acid position 43 of the SETDB1 protein MBD domain, which is optionally citrullinated.

In some embodiments, the polypeptide and variant thereof further comprises an arginine residue at amino acid position corresponding to the position 50 of the SETDB1 protein MBD domain, which is optionally citrullinated.

In some embodiments, the isolated polypeptide is composed of no more than 72 (e.g., no more than 70, no more than 69, no more than 68, no more than 67, no more than 66, no more than 65, no more than 64, no more than 63, no more than 62, no more than 61, no more than 60, no more than 59, no more than 58, no more than 57, no more than 56, no more than 55, no more than 54, no more than 53, no more than 52, no more than 51, no more than 50, no more than 49, no more than 48, no more than 47, no more than 46, no more than 45, no more than 44, no more than 43, no more than 42, no more than 41, no more than 40, no more than 39, no more than 38, no more than 37, no more than 36, no more than 35, no more than 34, no more than 33, no more than 32, no more than 31, no more than 30, no more than 29, or no more than 28) consecutive amino acid residues from the MBD domain of SETDB1.

In certain embodiments, the isolated polypeptide comprises 11 to 30 (e.g., 11 to 28) consecutive amino acid residues of the SETDB1 protein MBD domain. In certain embodiments, the isolated polypeptide is composed of 11, 12, 14, 16, 20, 23, 27, or 28 consecutive amino acid residues of the MBD domain of SETDB1.

In certain embodiments, the polypeptide and variant thereof of the present invention comprise at least amino acid residues at the positions 2 to 26 of the SETDB1 protein MBD domain.

In some embodiments, the isolated polypeptide comprises arginine residues at amino acid positions corresponding to the positions 2, 16, 17, 21, 22, 23, and 26 of the SETDB1 protein MBD domain, and at least one (e.g., 1, 2, 3, 4, 5, 6, or all 7) of these arginine residues is citrullinated.

In some embodiments, the isolated polypeptide is citrullinated at all arginine residues at positions corresponding to the positions 2, 16, 17, 21, 22, 23, and 26 of the SETDB1 protein MBD domain.

In some embodiments, the variant, compared to the polypeptide from which it is derived, does not comprise amino acid substitutions at positions corresponding to the following positions of the SETDB1 protein MBD domain: 2, 16, 17, 21, 22, 23, and 26. That is, the variant is identical to the polypeptide from which it is derived at amino acid positions corresponding to the positions 2, 16, 17, 21, 22, 23, and 26 of the SETDB1 protein MBD domain.

In some embodiments, the isolated polypeptide and variant thereof is composed of no more than 50 (e.g., no more than 49, no more than 48, no more than 47, no more than 46, no more than 45, no more than 44, no more than 43, no more than 42, no more than 41, no more than 40, no more than 39, no more than 38, no more than 37, no more than 36, no more than 35, no more than 34, no more than 33, no more than 32, no more than 31, or no more than 30) consecutive amino acid residues of the SETDB1 protein MBD domain.

In some embodiments, the isolated polypeptide and variant thereof is composed of 25 to 30 consecutive amino acid residues of the SETDB1 protein MBD domain.

In some embodiments, the SETDB1 protein MBD domain has a sequence as set forth in SEQ ID NO: 1, or a sequence having a sequence identity of at least 80%, at least 85%, at least 90%, at least 95%, at least 98%, or at least 99% as compared thereto.

In some embodiments, the isolated polypeptide has a sequence as set forth in any one of SEQ ID NOs: 3-10. In some embodiments, the variant has a sequence identity of at least 80%, at least 85%, at least 90%, at least 95%, at least 98%, or at least 99% as compared with the sequence as set forth in any one of SEQ ID NOs: 3-10, and has exactly the same citrulline positions and number.

### Derivatized Polypeptide or Variant thereof

The polypeptide or variant thereof of the present invention can be derivatized, for example, by linking other functional unit, such as detectable label, protein tag, solid-phase support, etc.

In one embodiment, the isolated polypeptide or variant thereof is attached to the surface of a solid-phase support or has a modifying group that can be attached to a solid-phase support. In some embodiments, the N-terminus of the isolated polypeptide or variant thereof is attached to the surface of a solid-phase support or has a modifying group that can be attached to a solid-phase support. In some embodiments, the modifying group is biotin or avidin. In some embodiments, the solid-phase support is selected from the group consisting of magnetic beads or microtiter plates (e.g., microplates or ELISA plates).

In another embodiment, the isolated polypeptide or variant thereof bears a detectable label. In some embodiments, the detectable label is selected from the group consisting of enzymes (e.g., horseradish peroxidase or alkaline phosphatase), chemiluminescent reagents (e.g., acridinium ester compounds), fluorescent dyes, or biotin.

### Preparation of Polypeptide

The polypeptide or variant thereof of the present invention is not limited in its production method; for example, it can be produced by a genetic engineering method (recombinant technology) or by a chemical synthesis method.

In another aspect, the present invention provides an isolated nucleic acid molecule, which comprises a nucleotide sequence encoding the polypeptide or variant thereof of the present invention.

In another aspect, the present invention also provides a vector, which comprises the isolated nucleic acid molecule as described above. The vector of the present invention can be a cloning vector or an expression vector. In a preferred embodiment, the vector of the present invention is, for example, a plasmid, a phage, a cosmid, etc.

In another aspect, the present invention also provides a host cell, which comprises the isolated nucleic acid molecule or vector of the present invention. Such host cell includes, but is not limited to, prokaryotic cell such as *E. coli* cell, eukaryotic cell such as yeast cell, insect cell, plant cell, and animal cell (e.g., mammalian cell, such as mouse cell, human cell, etc.).

In another aspect, the present invention also provides a method for preparing the polypeptide or variant thereof of the present invention, comprising culturing the host cell of the present invention under conditions allowing expression of the polypeptide or variant thereof, and recovering the polypeptide or variant thereof from a culture of the cultured host cell.

### Kit

In a second aspect, the present invention provides a kit, which comprises the isolated polypeptide or variant thereof is selected from those described in the first aspect.

In some embodiments, the kit of the present invention comprises a capture reagent, in which the capture reagent is selected from the isolated polypeptide or variant thereof of the first aspect. In some embodiments, the kit further comprises an instruction for using the isolated polypeptide or variant thereof as a capture reagent to detect an antibody specific to citrullinated SETDB1 protein in a sample, or an instruction for using the isolated polypeptide or variant thereof as a capture reagent to determine the level of an antibody specific to citrullinated SETDB 1 protein in a sample from a subject, thereby determining whether the subject has rheumatoid arthritis.

In some embodiments, the subject is a mammal, such as a human.

In some embodiments, the capture reagent is attached to the surface of a solid-phase support. In some embodiments, the N-terminus of the capture reagent is attached to the solid-phase support. As used herein, the solid-phase support includes well plates, tube, beads (e.g., latex particles), or films (e.g., nitrocellulose membrane) made of or coated with polymeric materials (e.g., polyvinyl chloride, polystyrene, polyacrylamide, or cellulose), or magnetic beads pre-coated with functional groups (e.g., amino, carboxyl, biotin, or avidin). In some embodiments, the solid-phase support is selected from magnetic beads or microtiter plates (e.g., microplates or ELISA plates).

In other embodiments, the capture reagent has a modifying group that can be attached to the solid-phase support. In some embodiments, the N-terminus of the capture reagent has a modifying group that can be attached to the solid-phase support. In such embodiments, the kit may further comprise a coating reagent, such as a coating buffer (e.g., carbonate buffer, phosphate buffer, Tris-HCl buffer, or borate buffer), for coating the capture reagent onto the solid-phase support. Methods for coating proteins or polypeptides onto a solid-phase support are well known in the art, such as physical adsorption, covalent coupling via aminated or carboxylated surfaces, or mediated binding via avidin-biotin systems, polylysine pre-coated surfaces, protein A or protein G pre-coated surfaces. In some embodiments, the modifying group is biotin or avidin, and the solid-phase support surface has a corresponding linker group.

In some embodiments, the kit contains at least a solid-phase support coated with avidin or streptavidin in a separate container or in a separate compartment of a single container unit, and the aforementioned capture reagent.

In some embodiments, the method for determining whether a subject has rheumatoid arthritis by measuring the level of anti-citrullinated SETDB1 antibody in a sample from a subject is performed in an indirect manner. Measuring antibody levels in a sample indirectly is well known to those skilled in the art. In such assays, a "capture antigen" first forms an immune complex with an antibody in the sample, and the captured antibody is subsequently detected by a secondary antibody (e.g., anti-immunoglobulin antibody).

Therefore, in some embodiments, the kit further comprises a detection reagent, in which the detection reagent is selected from an secondary antibody with a detectable label. In some embodiments, the detectable label is selected from enzymes (e.g., horseradish peroxidase or alkaline phosphatase), chemiluminescent reagents (e.g., acridine esters), fluorescent dyes, or biotin. In some exemplary embodiments, the detectable label is selected from enzymes (e.g., horseradish peroxidase or alkaline phosphatase).

In some embodiments, the secondary antibody is specific to the species from which the antibody ti be tested originates (e.g., human).

In some embodiments, the secondary antibody is an anti-immunoglobulin antibody.

In some exemplary embodiments, the kit is used to detect an anti-citrullinated SETDB1 protein IgG antibody. In such embodiments, the anti-immunoglobulin antibody is selected from anti-IgG antibodies, such as anti-human IgG antibodies.

In some exemplary embodiments, the kit is used to detect an anti-citrullinated SETDB1 protein IgM antibody. In such embodiments, the anti-immunoglobulin antibody is selected from anti-IgM antibodies, such as anti-human IgM antibodies.

In some exemplary embodiments, the kit is used to detect an anti-citrullinated SETDB1 protein IgA antibody. In such embodiments, the anti-immunoglobulin antibody is selected from anti-IgA antibodies, such as anti-human IgA antibodies.

In other embodiments, the method of determining the level of an anti-citrullinated SETDB1 antibody in a sample from a subject to determine whether the subject has rheumatoid arthritis is performed in a double-antigen sandwich configuration. Determining antibody level in a sample using a double-antigen sandwich method is well known to those skilled in the art. In such assays, "capturing antigen" and "detection antigen" cause the sample antibody to form a bridge between two specific antigens, and thus the two antigens are typically identical, or have the same core epitope, or have immune cross-reactivity, allowing one antibody to bind to both antigens.

Therefore, in some embodiments, the kit further comprises a detection reagent, in which the detection reagent is selected from the isolated polypeptide or variant thereof as described in the first aspect.

In some embodiments, the detection reagent bears a detectable label. In some embodiments, the detectable label is selected from enzymes (e.g., horseradish peroxidase or alkaline phosphatase), chemiluminescent reagents (e.g., acridinium ester compounds), fluorescent dyes, or biotin. In some exemplary embodiments, the detectable label is selected from enzymes (e.g., horseradish peroxidase or alkaline phosphatase).

In some embodiments, the polypeptide sequences contained in the detection reagent and the capture reagent are identical or substantially identical. In some embodiments, "substantially identical" means that the two polypeptide sequences contained in the detection reagent and the capture reagent comprise the same core epitope, in which the core epitope comprises at least the consecutive amino acid residues at the positions 16 to 26 of the SETDB1 protein MBD domain, at least one of the consecutive amino acid residues is an arginine residue, and at least one of the arginine residues is citrullinated (preferably all citrullinated). In some embodiments, the core epitope comprises at least the sequence as set forth in SEQ ID NO: 8.

In some embodiments, the kit of the present invention may also comprise one or more reagents or devices selected from: (i) a device for collecting or storing the sample from the subject (e.g., a blood collection device); (ii) other reagents required for performing the assay (e.g., buffers, diluents, blocking solutions, and/or standards).

### Detection Use and Method

In a third aspect, the present invention provides a method for detecting an antibody specific to citrullinated SETDB1 protein in a sample, comprising the following steps:
(1) contacting the sample with a capture reagent to obtain an antigen-antibody immune complex; in which the capture reagent is selected from the isolated polypeptide or variant thereof as described in the first aspect;
(2) determining the amount of the antigen-antibody immune complex obtained in step (1).

In some embodiments, the method is performed in vitro.

In some embodiments, the method is used for a non-diagnostic purpose.

In some embodiments, in step (2), the amount of the immune complex is determined by immunological detection.

In some embodiments, the immunological detection is selected from enzyme immunoassay (e.g., ELISA), chemiluminescent immunoassay, fluorescence immunoassay, or radioimmunoassay.

In some embodiments, the capture reagent is attached to the surface of a solid-phase support.

In other embodiments, the capture reagent has a modifying group capable of attaching to the solid-phase support. In such embodiments, prior to step (1), the method further comprises a step of coating the capture reagent onto the surface of the solid-phase support.

In some embodiments, the N-terminus of the capture reagent is attached to the surface of the solid-phase support, or has a modifying group capable of attaching to the solid-phase support.

In some embodiments, the determination is performed indirectly. Determining antibody level in a sample indirectly is well known to those skilled in the art. In such determination, the "capture antigen" first forms an immune complex with an antibody in the sample, and then the captured antibody is detected by a secondary antibody (e.g., anti-immunoglobulin antibody).

Therefore, in some embodiments, in step (2), a detection reagent is used to detect the amount of the immune complex, in which the detection reagent is selected from secondary antibodies bearing a detectable label.

In some embodiments, the detectable label is selected from enzymes (e.g., horseradish peroxidase or alkaline phosphatase), chemiluminescent reagents (e.g., acridinium ester compounds), fluorescent dyes, or biotin.

In some embodiments, the secondary antibody is specific to an antibody from the species (e.g., humans) from which the antibody to be detected is derived.

In some embodiments, the secondary antibody is an anti-immunoglobulin antibody.

In some exemplary embodiments, the antibody to be detected is an IgG antibody. In such embodiments, the anti-immunoglobulin antibody is selected from anti-IgG antibodies, such as anti-human IgG antibodies.

In some exemplary embodiments, the antibody to be detected is an IgM antibody. In such embodiments, the anti-immunoglobulin antibody is selected from anti-IgM antibodies, such as anti-human IgM antibodies.

In some exemplary embodiments, the antibody to be detected is an IgA antibody. In such embodiments, the anti-immunoglobulin antibody is selected from anti-IgA antibodies, such as anti-human IgA antibodies.

In other embodiments, the determination is performed in a double-antigen sandwich configuration. Determining antibody level in a sample using a double-antigen sandwich method is well known to those skilled in the art. In such determination, the "capture antigen" and "detect antigen" allow the sample antibody to form a bridge between two specific antigens, and thus the two antigens are typically identical, or have the same core epitope, or have immune cross-reactivity, allowing one antibody to bind to both antigens.

Therefore, in some embodiments, in step (2), a detection reagent is used to detect the amount of the immune complex, in which the detection reagent is selected from the isolated polypeptide or variant thereof as described in the first aspect.

In some embodiments, the detection reagent is selected from the isolated polypeptide or variant thereof as described in the first aspect, and the isolated polypeptide or variant thereof bears a detectable label. In some embodiments, the detectable label is selected from enzymes (e.g., horseradish peroxidase or alkaline phosphatase), chemiluminescent reagents (e.g., acridinium ester compounds), fluorescent dyes, or biotin. In some exemplary embodiments, the detectable label is selected from enzymes (e.g., horseradish peroxidase or alkaline phosphatase).

In some embodiments, the polypeptide sequences contained in the detection reagent and the capture reagent are identical or substantially identical. In some embodiments, "substantially identical" means that the two polypeptide sequences contained in the detection reagent and the capture reagent comprise the same core epitope, in which the core epitope comprises at least the consecutive amino acid residues at the positions 16 to 26 of the SETDB1 protein MBD domain, at least one of the consecutive amino acid residues is an arginine residue, and at least one of the arginine residues is citrullinated (preferably all citrullinated). In some embodiments, the core epitope comprises at least the sequence as set forth in SEQ ID NO: 8.

In another aspect, the present invention also relates to the use of the isolated polypeptide or variant thereof as described in the first aspect in the manufacture of a kit for detecting an antibody specific to a citrullinated SETDB 1 protein in a sample. In some embodiments, the kit detects the antibody specific to the citrullinated SETDB 1 protein in the sample by the method as described in the third aspect.

### Diagnostic Use and Method

In a fourth aspect, the invention provides a method for determining whether a subject has rheumatoid arthritis, comprising:
(1) measuring the level of an antibody specific to citrullinated SETDB1 protein in a sample from the subject; and,
(2) comparing the level with a reference value.

In some embodiments, if the level is higher than the reference value, the subject is determined to have rheumatoid arthritis.

In some embodiments, the method is performed in vitro.

As used herein, the reference value is a value derived from a subject without rheumatoid arthritis or a healthy person (e.g., a subject who has no detectable disease and no history of rheumatoid arthritis), or an indicator of the level of the antibody specific to citrullinated SETDB 1 protein in a corresponding sample from a subject without rheumatoid arthritis or a healthy person.

In some embodiments, the sample is a blood sample (e.g., whole blood, plasma, or serum) or a tissue fluid sample (e.g., synovial fluid).

In some embodiments, the subject is a mammal, such as a human.

In some embodiments, the level of the antibody specific to citrullinated SETDB 1 protein in the sample is determined by an immunoassay. In some embodiments, the immunoassay is selected from enzyme immunoassay (e.g., ELISA), chemiluminescent immunoassay, fluorescence immunoassay, or radioimmunoassay.

In some embodiments, the determination comprises using the isolated polypeptide or variant thereof as described in the first aspect as a capture reagent.

In some embodiments, step (1) comprises the following steps:
(1a) contacting a sample from the subject with a capture reagent to obtain an antigen-antibody immune complex; in which the capture reagent is selected from the isolated polypeptide or variant thereof as described in the first aspect;
(1b) determining the amount of the antigen-antibody immune complex obtained in step (1a).

In some embodiments, in step (1b), the amount of the immune complex is determined by an immunological assay. In some embodiments, the immunological assay is selected from enzyme immunoassay (e.g., ELISA), chemiluminescent immunoassay, fluorescence immunoassay, or radioimmunoassay.

In some embodiments, the capture reagent is attached to the surface of a solid-phase support.

In other embodiments, the capture reagent has a modifying group capable of attaching to the solid-phase support. In such embodiments, prior to step (1), the method further comprises a step of coating the capture reagent onto the surface of the solid-phase support.

In some embodiments, the N-terminus of the capture reagent is attached to the surface of the solid-phase support, or has a modifying group capable of attaching to the solid-phase support.

In some embodiments, the determination is performed in an indirect manner. Determining antibody level in a sample using an indirect method is well known to those skilled in the art. In such determination, a "capture antigen" first forms an immune complex with an antibody in the sample, and the captured antibody is then detected by a secondary antibody (e.g., an anti-immunoglobulin antibody).

Therefore, in some embodiments, in step (1b), a detection reagent is used to detect the amount of the immune complex, in which the detection reagent is selected from secondary antibodies bearing a detectable label.

In some embodiments, the detectable label is selected from enzymes (e.g., horseradish peroxidase or alkaline phosphatase), chemiluminescent reagents (e.g., acridinium ester compounds), fluorescent dyes, or biotin.

In some embodiments, the secondary antibody is specific to the species (e.g., human) from which the antibody to be detected is derived.

In some embodiments, the secondary antibody is an anti-immunoglobulin antibody.

In some exemplary embodiments, the antibody to be detected is an IgG antibody. In such embodiments, the anti-immunoglobulin antibody is selected from anti-IgG antibodies, such as anti-human IgG antibodies.

In some exemplary embodiments, the antibody to be detected is an IgM antibody. In such embodiments, the anti-immunoglobulin antibody is selected from anti-IgM antibodies, such as anti-human IgM antibodies.

In some exemplary embodiments, the antibody to be detected is an IgA antibody. In such embodiments, the anti-immunoglobulin antibody is selected from anti-IgA antibodies, such as anti-human IgA antibodies.

In other embodiments, the determination is performed in a double-antigen sandwich configuration. Determining antibody level in a sample using a double-antigen sandwich method is well known to those skilled in the art. In such determination, "capture antigen" and "detection antigen" cause the sample antibody to form a bridge between two specific antigens, and thus the two antigens are typically identical, or have the same core epitope, or have immune cross-reactivity, allowing one antibody to bind to both antigens.

Therefore, in some embodiments, in step (1b), a detection reagent is used to detect the amount of the immune complex, in which the detection reagent is selected from the isolated polypeptide or variant thereof as described in the first aspect.

In some embodiments, the detection reagent is selected from the isolated polypeptide or variant thereof as described in the first aspect, and the isolated polypeptide or variant thereof bears a detectable label. In some embodiments, the detectable label is selected from enzymes (e.g., horseradish peroxidase or alkaline phosphatase), chemiluminescent reagents (e.g., acridinium ester compounds), fluorescent dyes, or biotin. In some exemplary embodiments, the detectable label is selected from enzymes (e.g., horseradish peroxidase or alkaline phosphatase).

In some embodiments, the polypeptide sequences contained in the detection reagent and the capture reagent are identical or substantially identical. In some embodiments, "substantially identical" means that the two polypeptide sequences contained in the detection reagent and the capture reagent comprise the same core epitope, in which the core epitope comprises at least the consecutive amino acid residues at the positions 16 to 26 of the SETDB1 protein MBD domain, at least one of the consecutive amino acid residues is an arginine residue, and at least one of the arginine residues is citrullinated (preferably all citrullinated). In some embodiments, the core epitope comprises at least the sequence as set forth in SEQ ID NO: 8.

In some embodiments, the method described in any of the above embodiments further comprises: before step (1), providing a sample from the subject.

In some embodiments, the method described in any of the above embodiments further comprises, after step (2), administering a therapeutically effective amount of a therapy for rheumatoid arthritis (e.g., chemotherapy, radiotherapy, and/or immunotherapy) to a subject diagnosed with rheumatoid arthritis.

In some embodiments, therapy for rheumatoid arthritis is selected from the group consisting of surgical treatment, chemotherapy (e.g., hydroxychloroquine sulfate, sulfasalazine, leflunomide, methotrexate, prednisone, celecoxib, ibuprofen, diclofenac sodium, loxoprofen sodium), targeted therapy (e.g., tumor necrosis factor antagonist), immunotherapy (e.g., tocilizumab), and combination therapy (e.g., chemotherapy + surgery).

In another aspect, the invention also relates to the use of a reagent capable of determining an antibody specific to citrullinated SETDB1 protein in the manufacture of a kit for determining whether a subject has rheumatoid arthritis.

In some embodiments, the reagent is capable of determining the level of the antibody specific to citrullinated SETDB 1 protein by an immunological assay. In some embodiments, the immunological assay is selected from enzyme immunoassay (e.g., ELISA), chemiluminescent immunoassay, fluorescence immunoassay, or radioimmunoassay.

In some embodiments, the subject is a mammal, such as a human.

In some embodiments, the sample is a blood sample (e.g., whole blood, plasma, or serum) or a tissue fluid sample (e.g., synovial fluid).

In some embodiments, the kit determines whether a subject has rheumatoid arthritis by the following method:
(1) determining the level of the antibody specific to citrullinated SETDB1 protein in a sample from the subject; and,
(2) comparing the level to a reference value.

In some embodiments, if the level is higher than the reference value, the subject is determined to have rheumatoid arthritis.

In some embodiments, the reagent capable of determining the antibody specific to citrullinated SETDB 1 protein comprises a capture reagent, in which the capture reagent is selected from the isolated polypeptide or variant thereof as described in the first aspect.

In some embodiments, step (1) above comprises the following steps:
(1a) contacting the sample from the subject with a capture reagent to obtain an antigen-antibody immune complex; in which the capture reagent is selected from the isolated polypeptide or variant thereof as described in the first aspect;
(1b) determining the amount of the antigen-antibody immune complex obtained in step (1a).

In some embodiments, in step (1b), the amount of the immune complex is determined by an immunological assay. In some embodiments, the immunological assay is selected from enzyme immunoassay (e.g., ELISA), chemiluminescent immunoassay, fluorescence immunoassay, or radioimmunoassay.

In some embodiments, the capture reagent is attached to the surface of a solid-phase support.

In other embodiments, the capture reagent has a modifying group capable of attaching to the solid-phase support. In such embodiments, prior to step (1) above, the method further comprises a step of coating the capture reagent onto the surface of the solid-phase support.

In some embodiments, the N-terminus of the capture reagent is attached to the surface of the solid-phase support, or has a modifying group capable of attaching to the solid-phase support.

In some embodiments, the reagent capable of determining the antibody specific to citrullinated SETDB1 protein further comprises a detection reagent selected from secondary antibodies bearing a detectable label.

In some embodiments, in step (1b) above, the detection reagent is used to detect the amount of the immune complex.

In some embodiments, the detectable label is selected from enzymes (e.g., horseradish peroxidase or alkaline phosphatase), chemiluminescent reagents (e.g., acridinium ester compounds), fluorescent dyes, or biotin.

In some embodiments, the secondary antibody is specific to the species (e.g., human) from which the antibody to be detected is derived.

In some embodiments, the secondary antibody is an anti-immunoglobulin antibody.

In some exemplary embodiments, the antibody to be detected is an IgG antibody. In such embodiments, the anti-immunoglobulin antibody is selected from anti-IgG antibodies, such as anti-human IgG antibodies.

In some exemplary embodiments, the antibody to be detected is an IgM antibody. In such embodiments, the anti-immunoglobulin antibody is selected from anti-IgM antibodies, such as anti-human IgM antibodies.

In some exemplary embodiments, the antibody to be detected is an IgA antibody. In such embodiments, the anti-immunoglobulin antibody is selected from anti-IgA antibodies, such as anti-human IgA antibodies.

In other embodiments, the reagent capable of detecting the antibody specific to citrullinated SETDB 1 protein further comprises a detection reagent, in which the detection reagent is selected from the isolated polypeptide or variant thereof as described in the first aspect.

In some embodiments, in step (1b) above, the detection reagent is used to detect the amount of the immune complex.

In some embodiments, the detection reagent is selected from the isolated polypeptide or variant thereof as described in the first aspect, and the isolated polypeptide or variant thereof bears a detectable label. In some embodiments, the detectable label is selected from enzymes (e.g., horseradish peroxidase or alkaline phosphatase), chemiluminescent reagents (e.g., acridinium ester compounds), fluorescent dyes, or biotin. In some exemplary embodiments, the detectable label is selected from enzymes (e.g., horseradish peroxidase or alkaline phosphatase).

In some embodiments, the polypeptide sequences contained in the detection reagent and the capture reagent are identical or substantially identical. In some embodiments, "substantially identical" means that the two polypeptide sequences contained in the detection reagent and the capture reagent comprise the same core epitope, in which the core epitope comprises at least the consecutive amino acid residues at the positions 16 to 26 of the SETDB1 protein MBD domain, at least one of the consecutive amino acid residues is an arginine residue, and at least one of the arginine residues is citrullinated (preferably all citrullinated). In some embodiments, the core epitope comprises at least the sequence as set forth in SEQ ID NO: 8.

### Definition of Terms

In the present invention, unless otherwise stated, scientific and technical terms used herein have the meanings commonly understood by those skilled in the art. To better understand the present invention, definitions and explanations of relevant terms are provided below.

As used herein, the term "citrullination" refers to a form of post-translational modification (PTM), specifically, a chemical change resulting from the deiminolation of the amino acid arginine (R) to citrulline (C) catalyzed by peptidyl arginine deiminase (PAD), leading to the release of a nitrogen-containing moiety. In a citrullinated sequence, at least one arginine residue is substituted by a citrulline residue. Therefore, the expressions "arginine residue is citrullinated" and "arginine residue is substituted by a citrulline residue" have the same meaning.

As used herein, the terms "citrulline" and "cit" are used interchangeably and refer to 2-amino-5-(carbamoylamino)valerate, an α-amino acid having the formula: H₂NC(O)NH(CH₂)₃CH(NH₂)CO₂H.

As used herein, the term "anti-cyclic citrullinated peptide (CCP) antibody" refers to an autoantibody that uses a synthetic cyclic citrullinated peptide (CCP) as an antigen, primarily of the IgG type. Anti-CCP antibodies are spontaneously secreted by B lymphocytes in patients with rheumatoid arthritis, while B lymphocytes in patients with other diseases and healthy individuals do not spontaneously secrete anti-CCP antibodies. Therefore, anti-CCP antibodies have high specificity for rheumatoid arthritis.

As used herein, the term "SETDB1" refers to a histone methyltransferase that methylates the lysine residue at position 9 of histone H3 (H3K9) (Loyola A, et al., EMBO Reports.2009; 10(7):769-775; Gurard-Levin ZA, et al., Annu Rev Biochem.2014; 83:487-517). The human SETDB1 gene is located on human chromosome 1q21. The sequence of SETDB1 is well known to those skilled in the art; for example, the human SETDB1 gene sequence can be found in the Ensembl database accession number: ENSG00000143379. As used herein, "citrullinated SETDB1 protein" refers to a SETDB1 protein in which at least one arginine residue is substituted by a citrulline residue. Preferably, at least one arginine residue in the positions 16-26 of the MBD domain of the citrullinated SETDB1 protein is citrullinated (preferably all are citrullinated). Preferably, the positions 16-26 of the MBD domain of the citrullinated SETDB1 protein have the sequence as set forth in SEQ ID NO: 8.

As used herein, the term "SETDB1 protein MBD (methyl-CpG-binding domain)" refers to the methylated CpG-binding domain contained at the N-terminus of the SETDB1 protein, which is located at positions 594-665 of the SETDB1 protein. The sequence of the SETDB1 protein MBD domain is well known to those skilled in the art, and an exemplary sequence is provided in SEQ ID NO: 1.

As used herein, when referring to the amino acid sequence of the SETDB1 protein MBD domain, the sequence as set forth in SEQ ID NO: 1 is used for description. For example, the expression "amino acid residues at the positions 16-26 of the SETDB1 protein MBD domain" refers to the amino acid residues at the positions 16-26 of the polypeptide as set forth in SEQ ID NO: 1. However, those skilled in the art will understand that mutations or variations can be naturally generated or artificially introduced into the amino acid sequence of the SETDB1 protein MBD domain without affecting its biological function. Therefore, in the present invention, the term "SETDB1 protein MBD domain" and similar expressions should include all such sequences, including, for example, the sequence as set forth in SEQ ID NO: 1 and its natural or artificial variants (e.g., variants having a sequence identity of at least 80%, at least 85%, at least 90%, at least 95%, at least 98%, or at least 99%). Furthermore, when describing a sequence fragment of the SETDB1 protein MBD domain, it includes not only the sequence fragment of SEQ ID NO: 1 but also the corresponding sequence fragments in its natural or artificial variants. For example, the expression "amino acid residues at the positions 16-26 of the SETDB1 protein MBD domain" includes amino acid residues at the positions 16-26 of SEQ ID NO: 1, and the corresponding fragment in its (natural or artificial) variants. According to the present invention, the expression "corresponding sequence fragment" or "corresponding fragment" refers to a fragment located at an equivalent position in the compared sequences when the sequences are optimally aligned, i.e., when the sequences are aligned to obtain the highest percentage of identity.

As used herein, the terms "specifically bind to" or "specific to..." refer to a non-random binding reaction between two molecules (i.e., a binding molecule and a target molecule), such as the reaction between an antibody and its targeted antigen. The binding affinity between two molecules can be described by the KD value. The KD value is the dissociation constant obtained by the ratio of kd (the dissociation rate of a specific binding molecule-target molecule interaction; also known as koff) to ka (the association rate of a specific binding molecule-target molecule interaction; also known as kon), or kd/ka expressed as molar concentration (M). The smaller the KD value, the stronger the binding between the two molecules and the higher the affinity. In some embodiments, an antibody that specifically binds to an antigen (or an antibody that is specific to an antigen) means that the antibody binds to the antigen with an affinity (KD) of less than about 10⁻⁵ M, for example less than about 10⁻⁶ M, 10⁻⁷ M, 10⁻⁸ M, 10⁻⁹ M, or 10⁻¹⁰ M or less. The KD value can be determined by methods well known in the art, such as using surface plasmon resonance (SPR) in a BIACORE instrument.

As used herein, the term "immunological assay" refers to an assay that utilizes the specific interaction/binding affinity between an antigen and antibody, and is generally used to detect the presence or level of a specific antigen or antibody in a sample. Such immunological assays are well known to those skilled in the art and include, but are not limited to, enzyme immunoassay (EIA), chemiluminescent immunoassay (CLIA), radioimmunoassay (RIA), fluorescence immunoassay (FIA), Western blotting, immunoturbidimetry, surface plasmon resonance, etc. In some embodiments, the immunological assay is an enzyme immunoassay (EIA), such as an ELISA, Elispot assay, or CLEIA assay. For a detailed description of the immunological assay, see, for example, Fundamental Immunology, Ch. 7 Paul, W., ed., 2nd edition, Raven Press, N.Y. (1989).

As used herein, the term "epitope" refers to a site on an antigen that is specifically bound by an immunoglobulin or antibody. "Epitope" is also referred to in the art as an "antigenic determinant." For example, an epitope typically comprises at least 3, 4, 5, 6, 7, 8, 9, 10, 11, 12, 13, 14, or 15 consecutive or non-consecutive amino acids in a distinctive spatial conformation, and can be "linear" or "conformational." In a linear epitope, all points of interaction between the protein and the interacting molecule (e.g., an antibody) are present linearly along the primary amino acid sequence of the protein. In a conformational epitope, points of interaction are present across protein amino acid residues that are separated from each other.

As used herein, the term "detectable label" can refer to any substance that can be detected by fluorescent, spectroscopic, photochemical, biochemical, immunological, electrical, optical, or chemical means. Particularly preferred are those labels that are suitable for immunological assays (e.g., enzyme-linked immunosorbent assay, radioimmunoassay, fluorescence immunoassay, chemiluminescent immunoassay, etc.). Such labels are well known in the art and include, but are not limited to, enzymes (e.g., horseradish peroxidase, alkaline phosphatase, β-galactosidase, urease, glucose oxidase, etc.), radionuclides (e.g., ³H, ¹²⁵I, ³⁵S, ¹⁴C, or ³²P), fluorescent dyes (e.g., fluorescein isothiocyanate (FITC), fluorescein, tetramethylrhodamine isothiocyanate (TRITC), phycoerythrin (PE), Texas red, rhodamine, quantum dots, or cyanine dye derivatives (e.g., Cy7, Alexa 750)), luminescent substances (e.g., chemiluminescent substances, such as acridinium ester compounds), magnetic beads (e.g., Dynabeads^{®}), calorimetric labels such as colloidal gold or colored glass or plastic (e.g., polystyrene, polypropylene, latex, etc.) beads, and biotin for binding avidin (e.g., streptavidin) modified with the above labels. Patents teaching the use of these labels include, but are not limited to, U.S. Patents 3,817,837; 3,850,752; 3,939,350; 3,996,345; 4,277,437; 4,275,149; and 4,366,241 (all incorporated herein by reference). The labels covered in the present invention can be detected by methods known in the art. For example, radioactive labels can be detected using photographic film or a scintillation calculator, and fluorescent labels can be detected using a photodetector to detect emitted light. Enzyme labels are generally detected by providing an enzyme with a substrate and detecting the reaction product produced by the enzyme's action on the substrate, and calorimetric labels are detected by simple, visually stained labels. In some embodiments, the detectable labels described above can be linked to antibodies or antigens to be detected using linkers of varying lengths to reduce potential steric hindrance.

As used herein, the term "antibody" refers to an immunoglobulin molecule typically composed of two pairs of polypeptide chains, each pair having one light chain (LC) and one heavy chain (HC). Antibody light chains can be classified into κ (kappa) and λ (lambda) light chains. Heavy chains can be classified into µ, δ, γ, α, or ε, and antibody isotypes are defined as IgM, IgD, IgG, IgA, and IgE, respectively. Within both light and heavy chains, variable and constant regions are linked by a "J" region of approximately 12 or more amino acids, and the heavy chain also contains a "D" region of approximately 3 or more amino acids. Each heavy chain is composed of a heavy chain variable region (VH) and a heavy chain constant region (CH). The heavy chain constant region is composed of three domains (CH1, CH2, and CH3). Each light chain is composed of a light chain variable region (VL) and a light chain constant region (CL). The light chain constant region is composed of one domain, CL. Constant domains do not directly participate in antibody-antigen binding but exhibit various effector functions, such as mediating the binding of immunoglobulins to host tissues or factors, including various cells of the immune system (e.g., effector cells) and the first component (C1q) of the classical complement system. The VH and VL regions can be further subdivided into highly degenerated regions (called complementarity-determining regions (CDRs)) interspersed with more conservative regions called framework regions (FRs). Each VH and VL is composed of three CDRs and four FRs arranged in the following order: FR1, CDR1, FR2, CDR2, FR3, CDR3, FR4, from the amino terminus to the carboxyl terminus. The variable regions (VH and VL) of each heavy/light chain pair form the antigen-binding sites.

As used herein, the terms "isolated" or "being isolated" refer to those obtained artificially from their natural state. If an "isolated" substance or component appears in the nature, it may be due to an alteration of its natural environment, the isolation of the substance from its natural environment, or both. For example, a certain unisolated polynucleotide or polypeptide may naturally exist in a living animal, and a high-purity identical polynucleotide or polypeptide isolated from this natural state is called "isolated". The terms "isolated" or "being isolated" do not exclude the presence of an artificial or synthetic substance, nor do they exclude the presence of other impurities that do not affect the activity of the substance.

As used herein, the term "vector" refers to a nucleic acid delivery vehicle into which a polynucleotide can be inserted. When a vector enables the expression of the protein encoded by the inserted polynucleotide, the vector is called an expression vector. Vectors can be introduced into host cells through transformation, transduction, or transfection, allowing the genetic material elements they carry to be expressed in the host cells. Vectors are well-known to those skilled in the art and include, but are not limited to: plasmids; phage particles; cosmids; artificial chromosomes, such as yeast artificial chromosome (YAC), bacterial artificial chromosome (BAC), or P1-derived artificial chromosome (PAC); bacteriophages such as λ phage or M13 phage; and animal viruses. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, and papovavirus (e.g., SV40). A vector may contain multiple elements controlling expression, including but not limited to, promoter sequence, transcription initiation sequence, enhancer sequence, selection element, and reporter gene. Additionally, the vector may also contain a replication origin.

As used herein, the term "host cell" refers to any cell that can be used to introduce a vector, including but not limited to prokaryotic cell such as *Escherichia coli* or *Bacillus subtilis,* fungal cell such as yeast cell or *Aspergillus,* insect cell such as S2 *Drosophila* cell or Sf9, or animal cell such as fibroblast, CHO cell, COS cell, NSO cell, HeLa cell, BHK cell, HEK 293 cell, or human cell.

As used herein, the term "identity" refers to the sequence matching between two polypeptides or two nucleic acids. When a position in two compared sequences is occupied by the same base or amino acid monomer subunit (e.g., a position in each of two DNA molecules is occupied by adenine, or a position in each of two polypeptides is occupied by lysine), then the molecules are identical at that position. The "percentage identity" between two sequences is a function of the number of matching positions shared by the two sequences divided by the number of positions being compared × 100. For example, if six out of ten positions in two sequences match, then the two sequences have an identity of 60%. For example, the DNA sequences CTGACT and CAGGTT have an identity of 50% (three out of six positions match). Typically, two sequences are aligned to produce the maximum identity when they are compared. Such alignment can be achieved by the method of Needleman et al. (1970) J. Mol. Biol. 48:443-453, readily using, for example, computer programs such as the Align program (DNAstar, Inc.). The percentage identity between two amino acid sequences can also be determined using the algorithm of E. Meyers and W. Miller (Comput. Appl. Biosci., 4:11-17 (1988)), which has been integrated into the ALIGN program (version 2.0), with a PAM120 weight residue table, a gap length penalty of 12, and a gap penalty of 4. In addition, the percentage identity between two amino acid sequences can be determined using the algorithm of Needleman and Wunsch (J Mol Biol. 48:444-453 (1970)), which is integrated into the GAP program within the GCG software package (available at www.gcg.com), with the Blossum 62 matrix or PAM250 matrix, and gap weights of 16, 14, 12, 10, 8, 6, or 4, along with length weights of 1, 2, 3, 4, 5, or 6.

As used herein, the term "conservative substitution" refers to an amino acid substitution that does not adversely affect or alter the intended properties of a protein/peptide containing the amino acid sequence. For example, conservative substitutions can be introduced using standard techniques known in the art, such as site-directed mutagenesis and PCR-mediated mutagenesis. Conservative amino acid substitutions include substitutions of amino acid residues with amino acid residues having similar side chains, such as substitutions with residues that are physically or functionally similar to the corresponding amino acid residues (e.g., having similar size, shape, charge, chemical properties, including the ability to form covalent or hydrogen bonds). Families of amino acid residues with similar side chains have been defined in the art. These families include amino acids with basic side chains (e.g., lysine, arginine, and histidine), acidic side chains (e.g., aspartic acid and glutamic acid), uncharged polar side chains (e.g., glycine, asparagine, glutamine, serine, threonine, tyrosine, cysteine, and tryptophan), nonpolar side chains (e.g., alanine, valine, leucine, isoleucine, proline, phenylalanine, and methionine), β-branched side chains (e.g., threonine, valine, and isoleucine), and aromatic side chains (e.g., tyrosine, phenylalanine, tryptophan, and histidine). Therefore, it is preferable to replace the corresponding amino acid residue with another amino acid residue from the same side chain family. Furthermore, amino acid residues can also be classified into categories defined by optional physical and functional properties. For example, there are alcohol group-containing residues (S and T), aliphatic residues (I, L, V and M), cycloalkenyl-related residues (F, H, W and Y), hydrophobic residues (A, C, F, G, H, I, L, M, R, T, V, W and Y), negatively charged residues (D and E), polar residues (C, D, E, H, K, N, Q, R, S and T), positively charged residues (H, K and R), small residues (A, C, D, G, N, P, S, T and V), very small residues (A, G and S), residues involved in corner formation (A, C, D, E, G, H, K, N, Q, R, S, P and T), and flexible residues (Q, T, K, S, G, P, D, E and R). Methods for identifying conservative substitutions of amino acids are well known in the art (see, for example, Brummell et al., Biochem. 32:1180-1187 (1993); Kobayashi et al., Protein Eng. 12(10):879-884 (1999); and Burks et al., Proc. Natl Acad. Set USA 94:412-417 (1997), which are incorporated herein by reference).

The twenty common amino acids referred to herein are written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. Furthermore, in the present invention, amino acids are generally represented by single-letter and three-letter abbreviations well known in the art. For example, alanine can be represented as A or Ala.

As used herein, the term "subject" includes, but is not limited to, various animals, particularly mammals such as humans.

### Beneficial Effects

The present invention provides a citrullinated SETDB1 polypeptide and a method for detecting it based on anti-citrullinated SETDB1 antibody levels. The technical solution of the present invention has high specificity and sensitivity, enabling early diagnosis of rheumatoid arthritis. The technical solution of the present invention is simple to operate; compared to diagnosing patients through imaging, a preliminary diagnosis of disease can be made by detecting the level of anti-citrullinated SETDB1 antibody in the patient's serum. The technical solution of the present invention achieves a positive detection rate of up to 50% in individuals negative for anti-cyclic citrullinated peptide antibodies, greatly improving the detection rate, and solving the problem of missed diagnoses of early RA patients due to low sensitivity of diagnostic labels in existing technologies. Compared to artificially designed anti-CCP antibodies, the technical solution of the present invention involves a protein naturally present in the patient's body, which is more beneficial for discovering the pathogenesis of rheumatoid arthritis.

In summary, the citrullinated SETDB1 polypeptide and the detection method based on anti-citrullinated SETDB1 antibody levels provided by the present invention offer an effective solution to the problems of single diagnostic markers and easy missed diagnoses in existing technologies for RA, and are also of great significance for the study of the pathogenesis of RA.

### Brief Description of the Drawings

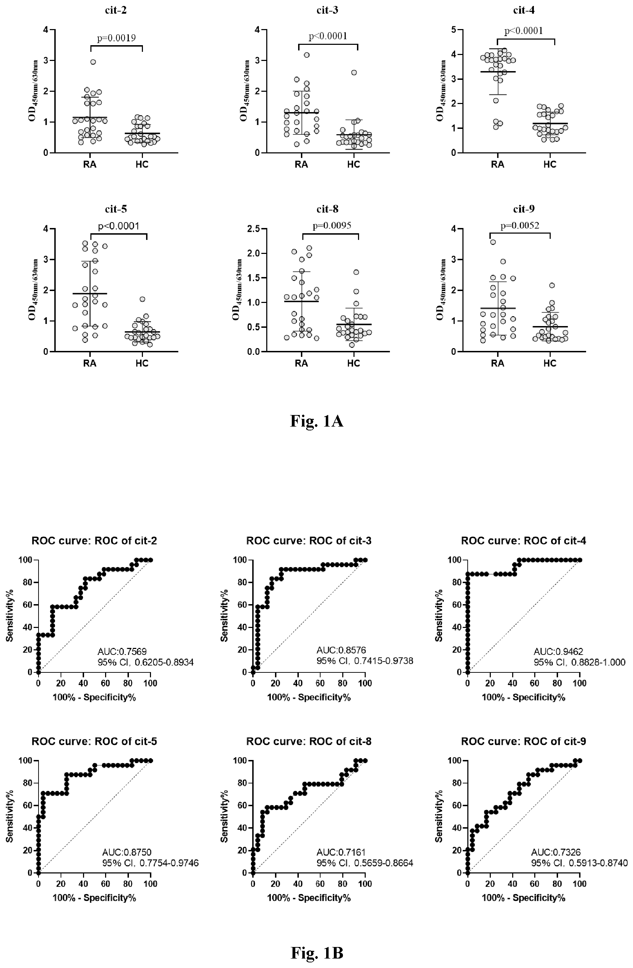

Figs. 1A to 1B show the antibody levels and diagnostic value assessment of different citrullinated short polypeptides in rheumatoid arthritis. Among which, Fig. 1A shows the antibody levels of different citrullinated short polypeptides in rheumatoid arthritis; and Fig. 1B shows the diagnostic value assessment of different citrullinated short polypeptides in rheumatoid arthritis.

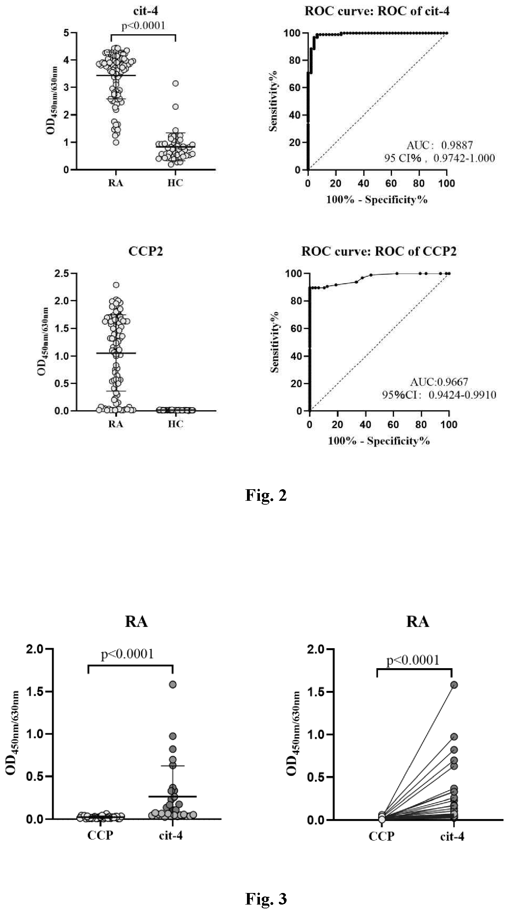

Fig. 2 shows the comparison of diagnostic value between citrullinated SETDB1 short polypeptide (cit-4) and anti-citrullinated peptide antibody (CCP2).
Fig. 3 shows the expression level study of citrullinated short polypeptide (cit-4) in anti-citrullinated peptide antibody (CCP2)-negative individuals.

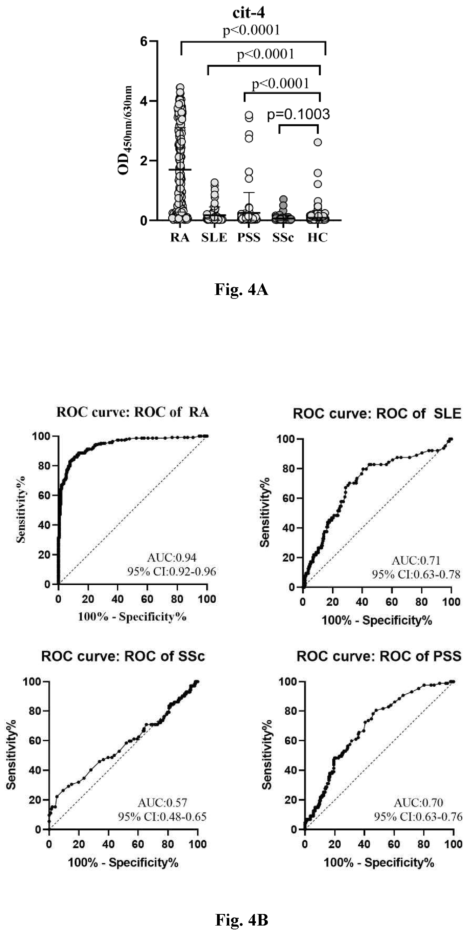

Figs. 4A to 4B show the antibody levels and diagnostic value study of citrullinated SETDB1 short polypeptide (cit-4) in patients with different autoimmune diseases. Among which, Fig. 4A shows the antibody levels of cit-4 in patients with different autoimmune diseases; and Fig. 4B shows the diagnostic value assessment of cit-4 in patients with different autoimmune diseases.

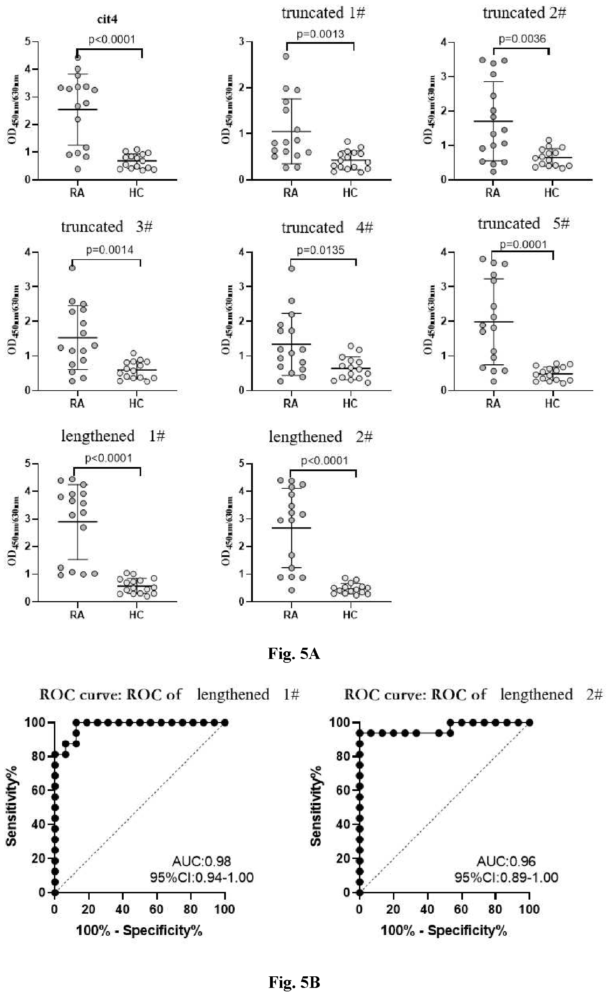

Figs. 5A to 5B show the detection results of SETDB1 citrulline polypeptides of different lengths in rheumatoid arthritis. Among which, Fig. 5A shows the antibody levels of SETDB1 citrulline polypeptides of different lengths in rheumatoid arthritis; and Fig. 5B shows the diagnostic value assessment of SETDB1 citrulline polypeptides of different lengths in rheumatoid arthritis.

### Sequence Information

**Table 1: Information on the sequences involved in the present invention is provided in the table below.**

| SEQ ID NO: | Description | Sequence |
|---|---|---|
| 1 | SETDB 1 MBD sequence | YRGKNPLLVPLLYDFRRMTARRRVNRKMGFH VIYKTPCGLCLRTMQEIERYLFETGCDFLFLEM FCLDPYVL |
| 2 | SETDB 1 -Arg-4 amino acid sequence (aa5-27) | NPLLVPLLYDFRRMTARRRVNRK |
| 3 | SETDB1 -cit-4 amino acid sequence (aa5-27) | NPLLVPLLYDFXXMTAXXXVNXK |
| 4 | SETDB1 -cit-4-truncated 1# amino acid sequence (aa8-27) | LVPLLYDFXXMTAXXXVNXK |
| 5 | SETDB1 -cit-4-truncated 2# amino acid sequence (aa12-27) | LYDFXXMTAXXXVNXK |
| 6 | SETDB1 -cit-4-truncated 3# amino acid sequence (aa14-27) | DFXXMTAXXXVNXK |
| 7 | SETDB1 -cit-4-truncated 4# amino acid sequence (aal6-27) | XXMTAXXXVNXK |
| 8 | SETDB1 -cit-4-truncated 5# amino acid sequence (aa16-26) | XXMTAXXXVNX |
| 9 | SETDB1 -cit-4-lengthened 1# amino acid sequence (aa1-27) | YXGKNPLLVPLLYDFXXMTAXXXVNXK |
| 10 | SETDB1 -cit-4-lengthened 2# amino acid sequence (aa5-32) | NPLLVPLLYDFXXMTAXXXVNXKMGFHV |
| 11 | cit-2 amino acid sequence | EVKGPSGXSFCCE |
| 12 | cit-3 amino acid sequence | NMSVTXSNSLXXDSPPPPARXAXQE |
| 13 | cit-5 amino acid sequence | PXMQIICXXXWXE |
| 14 | cit-8 amino acid sequence | ADMXXQMFAE |
| 15 | cit-9 amino acid sequence | MGPGDFXXCXE |

| | | |
|---|---|---|
| X represents citrulline. | | |

### Specific Models for Carrying Out the present invention

The present invention is now described in the following non-limiting examples.

Those skilled in the art will understand that the examples are described by way of example and are not intended to limit the scope sought to be protected by the present application. Unless otherwise specified, the experimental methods in the examples were conventional methods. Where specific conditions were not specified in the examples, they were performed under conventional conditions or conditions recommended by the manufacturer. Reagents or instruments whose manufacturers were not specified were all commercially available conventional products.

The ELISA detection reagents and consumables used in the following examples were donated by Beijing Wantai Biological Pharmacy Co., Ltd.; the detection antigens used were synthesized by Nanjing Genscript Biotech Co., Ltd.

### Example 1: Study on antibody level of citrullinated SETDB1 short polypeptide (cit-4) in rheumatoid arthritis

First, citrulline markers were screened from the serum of rheumatoid arthritis patients by mass spectrometry, initially obtaining citrulline short polypeptides including: cit-2 (SEQ ID NO: 11), cit-3 (SEQ ID NO: 12), cit-4 (SEQ ID NO: 3), cit-5 (SEQ ID NO: 13), cit-8 (SEQ ID NO: 14), and cit-9 (SEQ ID NO: 15). Subsequently, these short polypeptides were conjugated with biotin and chemically synthesized to provide biotin-labeled short polypeptides for further ELISA screening.

The autoantibody expression levels of the citrulline markers were detected using an indirect ELISA method. In short, biotin-conjugated short polypeptides were first coated onto PABC plates at a concentration of 50 ng/well. After coating, serum from 24 rheumatoid arthritis (RA) patients and 24 healthy subjects (HC) were diluted 10-fold as primary antibodies and incubated for 30 minutes. HRP-labeled goat anti-human IgG (GAH-HRP) was used as secondary antibody, incubation was performed for 30 minutes, and color development and reading were finally carried out. The results are shown in Fig. 1. Fig. 1A shows that the level of autoantibodies against cit-4 was significantly elevated in rheumatoid arthritis, with statistical significance. ROC curve analysis of the detection results was also performed, as shown in Fig. 1B, which indicated that cit-4 had high diagnostic value in rheumatoid arthritis, with an AUC value of 0.9462, significantly superior to other short polypeptides.

### Example 2: Study on diagnostic value of citrullinated SETDB1 short polypeptide (cit-4) for rheumatoid arthritis

The expression level of citrulline marker autoantibodies in patients with rheumatoid arthritis was detected using an indirect ELISA method (see Example 1 for details). In addition, anti-citrullinated peptide antibody (CCP2) was used as a control. The anti-citrullinated peptide antibody kit was purchased from Beijing Bell Biotechnology Co., Ltd., and the relevant assays were performed according to the kit instructions. The results are shown in Fig. 2. The results in Fig. 2 show that the AUC value of cit-4 was 0.9887, which was greater than that of CCP2 (AUC value is 0.9667), indicating a higher diagnostic efficacy compared to CCP2.

### Example 3: Study on expression level of citrullinated short polypeptide (cit-4) in anti-citrullinated peptide antibody (CCP2)-negative individuals

The cit-4 antibody level was detected in a CCP2-negative patient population using an indirect ELISA method (see Example 1 for details). The results are shown in Fig. 3. The results in Fig. 3 show that the positive rate of cit-4 in the CCP2-negative patient population could reach 50%, indicating that the sensitivity of cit-4 was superior to that of CCP2.

### Example 4: Study on diagnostic value of citrullinated SETDB1 short polypeptide (cit-4) in patients with different autoimmune diseases

The levels of citrullinated SETDB1 autoantibodies in the serum of patients with different autoimmune diseases (RA rheumatoid arthritis, SLE systemic lupus erythematosus, PSS primary Sjögren's syndrome, and SSc scleroderma) were detected using an indirect ELISA method (see Example 1 for details). The results are shown in Fig. 4. Fig. 4A shows that among autoimmune diseases, the level of citrullinated SETDB1 autoantibodies was the highest in rheumatoid arthritis. ROC curve analysis of the detection results was also performed, and the results are shown in Fig. 4B. Among autoimmune diseases, citrullinated SETDB1 showed the best diagnostic value for patients with rheumatoid arthritis.

### Example 5: Expression levels of citrullinated, truncated SETDB1 variants of different lengths in serum of patients with rheumatoid arthritis

Based on the citrullinated short polypeptide cit-4 (SEQ ID NO: 3), some citrullinated, truncated SETDB1 variants of different lengths (SEQ ID NOs: 4-10) were further designed and synthesized, all containing SEQ ID NO: 8 (i.e., amino acid residues at positions 16-26 of the MBD domain). These citrullinated, truncated SETDB1 variants (SEQ ID NOs: 3-10) were used to detect the expression levels of autoantibodies against citrullinated markers in rheumatoid arthritis patients, following the indirect ELISA method (see Example 1 for details). The results are shown in Fig. 5. Fig. 5A shows that these citrullinated, truncated SETDB1 polypeptides of different lengths showed significantly increased expression levels of autoantibodies in the serum of patients with rheumatoid arthritis. ROC curve analysis of the detection results was also performed, as shown in Fig. 5B, which indicated that these citrullinated, truncated SETDB1 polypeptides of different lengths could be used for the diagnosis of rheumatoid arthritis.

Although specific embodiments of the present invention have been described in detail, those skilled in the art will understand that various modifications and variations can be made to the details based on all the disclosed teachings, and these changes are all within the scope of protection of the present invention. The entire scope of the present invention is given by the appended claims and any equivalents thereof.

## Claims

1. An isolated polypeptide or variant thereof, wherein the polypeptide is composed of at least 11 consecutive amino acid residues of SETDB1 protein MBD domain;
wherein, at least one of the consecutive amino acid residues is an arginine residue, and at least one of the arginine residues is citrullinated;
wherein, the variant differs from the polypeptide from which it is derived only in a substitution (e.g., a conservative or non-conservative substitution) of one or more (e.g., 1, 2, 3, 4, 5, 6, 7, 8, or 9) amino acid residues, and retains a biological function (e.g., an activity of being recognized and bound by an anti-citrullinated SETDB1 antibody) of the polypeptide from which it is derived.

2. The isolated polypeptide or variant thereof according to claim 1, wherein the isolated polypeptide comprises an arginine residue at least at one of amino acid positions corresponding to the following positions of the SETDB1 protein MBD domain: 2, 16, 17, 21, 22, 23, 26, 43, and/or 50; and at least one of these arginine residues is citrullinated;
preferably, the isolated polypeptide comprises an arginine residue at least at one of amino acid positions corresponding to the following positions of the SETDB1 protein MBD domain: 16, 17, 21, 22, 23, and/or 26.

3. The isolated polypeptide or variant thereof according to claim 1 or 2, wherein the isolated polypeptide comprises at least amino acid residues at the positions 16-26 of the SETDB1 protein MBD domain; wherein at least one of the consecutive amino acid residues at the positions 16-26 is an arginine residue, and at least one of these arginine residues is citrullinated;
preferably, the isolated polypeptide comprises an arginine residue at least at one (e.g., 1, 2, 3, 4, 5, or all 6) of amino acid positions corresponding to the following positions of the SETDB1 protein MBD domain: 16, 17, 21, 22, 23, and/or 26; and at least one (e.g., 1, 2, 3, 4, 5, or all 6) of these arginine residues is citrullinated;
preferably, the isolated polypeptide comprises at least the sequence as set forth in SEQ ID NO: 8;
preferably, the isolated polypeptide further comprises an arginine residue at least at one (e.g., 1, 2, or all 3) of amino acid positions corresponding to the following positions of the SETDB1 protein MBD domain: 2, 43, and/or 50; and at least one (e.g., 1, 2, or all 3) of the arginine residues is citrullinated.

4. The isolated polypeptide or variant thereof according to any one of claims 1 to 3, wherein the isolated polypeptide is composed of consecutive amino acid residues selected from the following: amino acid residues at positions 5-27, 8-27, 12-27, 14-27, 16-27, 16-26, 1-27, or 5-32 of the SETDB1 protein MBD domain; wherein at least one of the consecutive amino acid residues is an arginine residue, and at least one of these arginine residues is citrullinated;
preferably, the isolated polypeptide comprises an arginine residue at least at one (e.g., 1, 2, 3, 4, 5, or all 6) of amino acid positions corresponding to the following positions of the SETDB1 protein MBD domain: 16, 17, 21, 22, 23, and/or 26; and at least one (e.g., 1, 2, 3, 4, 5, or all 6) of these arginine residues is citrullinated;
preferably, the isolated polypeptide further comprises an arginine residue at amino acid position corresponding to the position 2 of the SETDB1 protein MBD domain, which is optionally citrullinated.

5. The isolated polypeptide or variant thereof according to any one of claims 1 to 4, wherein the isolated polypeptide comprises no more than 72 (e.g., no more than 70, no more than 65, no more than 60, no more than 55, no more than 50, no more than 45, no more than 40, no more than 35, or no more than 30) consecutive amino acid residues of the SETDB1 protein MBD domain.

6. The isolated polypeptide or variant thereof according to any one of claims 1 to 5, wherein the variant does not comprise amino acid substitutions at amino acid positions corresponding to the following positions of the SETDB1 protein MBD domain: 16, 17, 21, 22, 23, and 26;
preferably, the variant further does not comprise amino acid substitutions at amino acid positions corresponding to the positions 2, 43, and/or 50 of the SETDB1 protein MBD domain.

7. The isolated polypeptide or variant thereof according to any one of claims 1 to 6, wherein the isolated polypeptide comprises at least amino acid residues at the positions 2-26 of the SETDB1 protein MBD domain;
preferably, the isolated polypeptide comprises an arginine residue at amino acid positions corresponding to the positions 2, 16, 17, 21, 22, 23, and 26 of the SETDB1 protein MBD domain, and at least one (e.g., 1, 2, 3, 4, 5, 6, or all 7) of these arginine residues is citrullinated;
preferably, all arginine residues at amino acid positions corresponding to the positions 2, 16, 17, 21, 22, 23, and 26 of the SETDB1 protein MBD domain are citrullinated.

8. The isolated polypeptide or variant thereof according to any one of claims 1 to 7, wherein the SETDB1 protein MBD domain has the sequence as set forth in SEQ ID NO: 1.

9. The isolated polypeptide or variant thereof according to any one of claims 1 to 8, wherein the isolated polypeptide has a sequence as set forth in any one of SEQ ID NOs: 3-10;
preferably, the variant has a sequence identity of at least 80%, at least 85%, at least 90%, at least 95%, at least 98%, or at least 99% as compared with the sequence as set forth in any one of SEQ ID NOs: 3-10, and has exactly the same citrulline positions and number.

10. The isolated polypeptide or variant thereof according to any one of claims 1 to 9, wherein the isolated polypeptide or variant thereof is attached to the surface of a solid-phase support or has a modifying group capable of attaching to the solid-phase support;
preferably, the modifying group is biotin or avidin;
preferably, the solid-phase support is selected from magnetic beads or microtiter plates (e.g., microplates or ELISA plates).

11. The isolated polypeptide or variant thereof according to any one of claims 1 to 9, wherein the isolated polypeptide or variant thereof bears a detectable label;
preferably, the detectable label is selected from enzymes (e.g., horseradish peroxidase or alkaline phosphatase), chemiluminescent reagents (e.g., acridinium ester compounds), fluorescent dyes, or biotin.

12. An isolated nucleic acid molecule, which comprises a nucleotide sequence encoding the isolated polypeptide or variant thereof according to any one of claims 1 to 9.

13. A vector, which comprises the isolated nucleic acid molecule according to claim 12.

14. A host cell, which comprises the isolated nucleic acid molecule according to claim 12 or the vector according to claim 13.

15. A kit, which comprises the isolated polypeptide or variant thereof according to any one of claims 1 to 9.

16. The kit according to claim 15, which comprises a capture reagent selected from the isolated polypeptide or variant thereof according to any one of claims 1 to 9;
preferably, the kit further comprises an instruction for using the isolated polypeptide or variant thereof as the capture reagent to determine the level of an antibody specific to citrullinated SETDB1 protein in a sample from a subject, and optionally to determine whether the subject has rheumatoid arthritis;
preferably, the capture reagent is selected from the isolated polypeptide or variant thereof according to claim 10;
preferably, the subject is a mammal, such as a human.

17. The kit according to claim 16, further comprising a detection reagent, wherein the detection reagent is selected from secondary antibodies bearing a detectable label;
preferably, the detectable label is selected from enzymes (e.g., horseradish peroxidase or alkaline phosphatase), chemiluminescent reagents (e.g., acridinium ester compounds), fluorescent dyes, or biotin;
preferably, the secondary antibody is specific to the species (e.g., human) from which the antibody to be detected is derived;
preferably, the secondary antibody is an anti-immunoglobulin antibody;
preferably, the anti-immunoglobulin antibody is selected from anti-IgG antibodies, anti-IgM antibodies, and anti-IgA antibodies.

18. The kit according to claim 16, further comprising a detection reagent, wherein the detection reagent is selected from the isolated polypeptide or variant thereof according to claim 11.

19. Use of a reagent capable of determining an antibody specific to citrullinated SETDB1 protein in the manufacture of a kit for determining whether a subject has rheumatoid arthritis;
preferably, the reagent is capable of determining the antibody specific to citrullinated SETDB1 protein by an immunological assay; preferably, the immunological assay is selected from enzyme immunoassay (e.g., ELISA), chemiluminescent immunoassay, fluorescence immunoassay, or radioimmunoassay;
preferably, the subject is a human;
preferably, the reagent is used to determine the level of the antibody specific to citrullinated SETDB1 protein in a sample from the subject;
preferably, the sample is a blood sample (e.g., whole blood, plasma, or serum) or a tissue fluid sample (e.g., synovial fluid).

20. The use according to claim 19, wherein the reagent capable of determining the antibody specific to citrullinated SETDB1 protein comprises a capture reagent, in which the capture reagent is selected from the isolated polypeptide or variant thereof according to any one of claims 1 to 7;
preferably, the capture reagent is selected from the isolated polypeptide or variant thereof according to claim 10.

21. The use according to claim 20, wherein the reagent capable of determining the antibody specific to citrullinated SETDB1 protein further comprises a detection reagent, in which the detection reagent is selected from secondary antibodies bearing a detectable label;
preferably, the detectable label is selected from enzymes (e.g., horseradish peroxidase or alkaline phosphatase), chemiluminescent reagents (e.g., acridinium ester compounds), fluorescent dyes, or biotin;
preferably, the secondary antibody is specific to the species (e.g., human) from which the antibody to be detected is derived;
preferably, the secondary antibody is an anti-immunoglobulin antibody;
preferably, the anti-immunoglobulin antibody is selected from anti-IgG antibodies, anti-IgM antibodies, or anti-IgA antibodies; preferably, the anti-immunoglobulin antibody is an anti-IgG antibody.

22. The use according to claim 20, wherein the reagent capable of determining the antibody specific to citrullinated SETDB1 protein further comprises a detection reagent, in which the detection reagent is selected from the isolated polypeptide or variant thereof according to claim 11.

23. Use of the isolated polypeptide or variant thereof according to any one of claims 1 to 11 in the manufacture of a kit for detecting an antibody specific to citrullinated SETDB1 protein in a sample.

24. A method for detecting an antibody specific to citrullinated SETDB1 protein in a sample, comprising the steps of:
(1) contacting the sample with a capture reagent to obtain an antigen-antibody immune complex; in which the capture reagent is selected from the isolated polypeptide or variant thereof according to any one of claims 1 to 9;
(2) determining the amount of the antigen-antibody immune complex obtained in step (1);
preferably, in step (2), the amount of the immune complex is determined by an immunological assay;
preferably, the immunological assay is selected from enzyme immunoassay (e.g., ELISA), chemiluminescent immunoassay, fluorescence immunoassay, or radioimmunoassay;
preferably, the capture reagent is selected from the isolated polypeptide or variant thereof according to claim 10;
preferably, the method is performed in vitro;
preferably, the method is for non-diagnostic purposes.

25. The method according to claim 24, wherein, in step (2), a detection reagent is used to detect the amount of the immune complex, in which the detection reagent is selected from secondary antibodies bearing a detectable label;
preferably, the detectable label is selected from enzymes (e.g., horseradish peroxidase or alkaline phosphatase), chemiluminescent reagents (e.g., acridinium ester compounds), fluorescent dyes, or biotin;
preferably, the secondary antibody is specific to the species (e.g., human) from which the antibody to be detected is derived;
preferably, the secondary antibody is an anti-immunoglobulin antibody;
preferably, the anti-immunoglobulin antibody is selected from anti-IgG antibodies, anti-IgM antibodies, or anti-IgA antibodies; preferably, the anti-immunoglobulin antibody is an anti-IgG antibody.

26. The method according to claim 24, wherein, in step (2), a detection reagent is used to detect the amount of the immune complex, in which the detection reagent is selected from the isolated polypeptide or variant thereof according to claim 11.

27. A method for determining whether a subject has rheumatoid arthritis, comprising:
(1) measuring the level of an antibody specific to citrullinated SETDB1 protein in a sample from the subject; and,
(2) comparing the level to a reference value;
preferably, if the level is higher than the reference value, the subject is determined to have rheumatoid arthritis;
preferably, the level of the antibody specific to citrullinated SETDB1 protein in the sample is determined by an immunological assay; preferably, the immunological assay is selected from enzyme immunoassay (e.g., ELISA), chemiluminescent immunoassay, fluorescence immunoassay, or radioimmunoassay;
preferably, the determination comprises using the isolated polypeptide or variant thereof according to any one of claims 1 to 11 as a capture reagent;
preferably, the sample is a blood sample (e.g., whole blood, plasma, or serum) or a tissue fluid sample (e.g., synovial fluid);
preferably, the subject is a mammal, such as a human.

28. The method according to claim 27, wherein step (1) comprises the following step:
(1a) contacting the sample from the subject with a capture reagent to obtain an antigen-antibody immune complex; in which the capture reagent is selected from the isolated polypeptide or variant thereof as described in the first aspect;
(1b) determining the amount of the antigen-antibody immune complex obtained in step (1a).

29. The method according to claim 27, wherein, in step (1b), the amount of the immune complex is determined by an immunological assay; preferably, the immunological assay is selected from enzyme immunoassay (e.g., ELISA), chemiluminescent immunoassay, fluorescence immunoassay, or radioimmunoassay.

30. The method according to claim 27, wherein, in step (1b), a detection reagent is used to detect the amount of the immune complex, in which the detection reagent is selected from secondary antibodies bearing a detectable label;
preferably, the detectable label is selected from enzymes (e.g., horseradish peroxidase or alkaline phosphatase), chemiluminescent reagents (e.g., acridinium ester compounds), fluorescent dyes, or biotin;
preferably, the secondary antibody is specific to the species (e.g., human) from which the antibody to be detected is derived;
preferably, the secondary antibody is an anti-immunoglobulin antibody;
preferably, the anti-immunoglobulin antibody is selected from anti-IgG antibodies, anti-IgM antibodies, or anti-IgA antibodies; preferably, the anti-immunoglobulin antibody is an anti-IgG antibody.

31. The method according to claim 27, wherein, in step (1b), a detection reagent is used to detect the amount of the immune complex, in which the detection reagent is selected from the isolated polypeptide or variant thereof according to any one of claims 1 to 11;
preferably, the isolated polypeptide or variant thereof bears a detectable label;
preferably, the detectable label is selected from enzymes (e.g., horseradish peroxidase or alkaline phosphatase), chemiluminescent reagents (e.g., acridinium ester compounds), fluorescent dyes, or biotin.
